(19) Europäisches Patentamt / European Patent Office / Office européen des brevets

(11) **EP 1 419 268 B1**

(12) **EUROPEAN PATENT SPECIFICATION**

(45) Date of publication and mention
of the grant of the patent:
**21.11.2012 Bulletin 2012/47**

(51) Int Cl.:
*C12Q 1/62* *(2006.01)*  *C12Q 1/34* *(2006.01)*
*A61B 10/00* *(2006.01)*  *A61F 13/42* *(2006.01)*

(21) Application number: **02751589.9**

(22) Date of filing: **18.07.2002**

(86) International application number:
**PCT/IL2002/000588**

(87) International publication number:
**WO 2003/007997 (30.01.2003 Gazette 2003/05)**

(54) **SECRETION-MONITORING ARTICLE**

ARTIKEL FÜR DIE SEKRETIONSÜBERWACHUNG

ARTICLE DE CONTROLE DE SECRETION

(84) Designated Contracting States:
**AT BE BG CH CY CZ DE DK EE ES FI FR GB GR IE IT LI LU MC NL PT SE SK TR**

(30) Priority: **19.07.2001 US 907926**
**18.03.2002 US 365684 P**

(43) Date of publication of application:
**19.05.2004 Bulletin 2004/21**

(73) Proprietor: **Common Sense Ltd.**
**Ceasare Industrial Park 38900 (IL)**

(72) Inventors:
• **KRITZMAN, Amnon**
**30900 Zichron Yaakov (IL)**
• **NACHSHON, Nitsa, G.**
**Geva 18915 (IL)**
• **BEHAR, Yael**
**Ein Ayala 30825 (IL)**

(74) Representative: **Becker Kurig Straus**
**Bavariastrasse 7**
**80336 München (DE)**

(56) References cited:
WO-A1-00/04822    WO-A1-01/13097
DE-U1- 29 514 562    GB-A- 2 353 357
US-A- 3 427 225    US-A- 3 427 225
US-A- 5 217 444    US-A- 5 468 236
US-A- 5 897 834

Description

## TECHNICAL FIELD

The present invention relates to the field of medical diagnostics and more specifically, to an improved identification of secreted biological fluids using a secretion-monitoring article to identify amniotic fluid or secretions associated with bacterial, fungal, or yeast infections even in the presence of interfering biological fluids. Improved methods of attaching an indicator to a substrate and methods of preparing and using a secretion-monitoring article to identify a secretion are also disclosed.

## BACKGROUND OF THE INVENTION

Many bodily fluids can be readily identified by chemical properties such as pH. One exceptionally useful method of determining the pH of a liquid sample is through the use of an indicator, a chemical compound or combination of compounds, that has a pH dependent color. Well known examples include tea and wine. General details and descriptions of some indicators can be found, for example, in "Indicators", E. Bishop, Pergamon Press, 1972, chapter 3.

Often an indicator is attached to a solid substrate such as paper. A sample of a liquid of which the pH needs to be determined is applied to the substrate. The pH of the liquid is determined by determining the color of the indicator present on the substrate. Depending on how the indicator is attached to the substrate, application of the liquid sample may cause the indicator to leach out of the substrate. Indicator leaching is undesirable and so the indicator is often substantially immobilized on the substrate.

Many medical conditions can be diagnosed by identifying the chemical and physical properties of a vaginal secretion, such as, by identifying the pH of the secretion. A number of devices involving panty shields with pH indicators are known in the art, for example in US Patents 5,217,444, 5,823,953 and 6,106,461. These devices can be worn by the user and whenever there is a secretion it s immediately detected by the pH indicator. International patent application WO01/13097, which discloses an indicator bound to a hydrophilic synthetic membrane substrate and a device, such as a panty shield with an indicator bound to hydrophilic synthetic membrane substrate.

A general problem, however, with these pH indicators is that they often provide "false positives" due to changes in pH on drying, interfering biological fluids and repetitive cycles of drying/wetting. Often a vaginal secretion cannot be identified with absolute certainty by an indicator due to the existence of a plurality of fluids collected with a similar pH. The "false positive" readings can be stressful and time consuming to the user. A device that minimizes these "false positive" readings is needed.

False positive readings can be caused, for example, by interfering biological fluids, such as urine. Vaginal secretions of a patient with vaginosis have a pH between 4.7 and 6.5. Because urine of a healthy patient has a pH between 5.0 and 8.0, it is very difficult to diagnose a secretion as arising from vaginosis with a high degree of confidence by just using a pH based indicator test. One solution known in the art is to sample fluid from within the vagina, where urine is not ordinarily found. This is uncomfortable and requires a visit to a health-care professional.

A second example is the identification of amniotic fluid leaking from the vagina of a pregnant woman. During pregnancy amniotic sac integrity may be compromised and a small amount of amniotic fluid may leak out through the cervix and from the vagina. If diagnosed as such, measures such as patient rest or sealing of the amniotic sack using biological glue may be prescribed. If not diagnosed the amniotic sack may later rupture causing abortion of the pregnancy, or require hospitalization of the woman and infant. If the infant is born prematurely, death or severe handicap may be a result. Extended hospitalization of the infant in an incubator is often necessary.

Due to the severe consequences of amniotic fluid leakage, pregnant women undergo severe stress and often go to a health-care professional upon secretion of any liquid from the vicinity of the vagina. The health-care professional looks for the presence of amniotic fluid by checking the pH of the vaginal secretions, amniotic fluid having a pH of between 6.0 and 8.0. Since pregnant women often have urinary incontinence and since urine typically has a pH of between 5.0 and 8.0, if only pH is checked, a false positive result may occur: urine being identified as amniotic fluid. Consequently, it is necessary that such a vaginal secretion be examined using a microscope for the presence of a fern-shaped pattern indicative of amniotic fluid.

As the time between the fluid secretion and the arrival at the health-care professional may be long, there is often no evidence of amniotic fluid upon examination. The secretion may mistakenly be assumed to be urine, often with tragic consequences. On the other hand, the healthcare professional may decide to err on the side of caution, misdiagnosing the secretion of urine as amniotic fluid leading to an unnecessary hospitalization and patient stress.

U.S. Patent No. 6,126,597 (the '597 patent) and U.S. Patent No. 6,149,590, (the '590 patent) a continuation-in-part of the '597 patent, are directed to a device in the form of a sanitary napkin with a pH indicator configured to identify the presence of amniotic fluid in a vaginal secretion is disclosed. The '597 and '590 patents are subject to the problem of giving false positive results. The device of the '590 patent address this problem by further including in the device a microscope visualizable slide configured to gather a portion of a vaginal secretion. If the indicator shows the pH corresponding to that of amniotic fluid, the user presents a health-care professional with the slide. The health-care professional examines the slide with the help of a microscope for the typical fern-shaped patterns indicative of the presence of amniotic fluid.

There are a couple of disadvantages associated with this device. First, it requires that the patient visit the health-care professional to distinguish between positive and false-positives and second, a significant amount of time is lost in the having the slide viewed by a professional to determine if amniotic fluid is actually leaking.

U.S. Patent No. 5,897,834 discloses a device useful in a clinical setting for the differentiation between urine and vaginal secretions associated with vaginosis or urine and amniotic fluid. The device includes the use of indicators with a negatively charged group immobilized to a solid polymer substrate containing quaternary ammonium groups. Further the device includes a gaseous amine-releasing reagent and an amine indicator. The use of the polymer substrate containing quaternary ammonium groups is disclosed to have an advantage of sharpening the pH dependent color transition. However, these polymer substrates have been found to be less useful in non-clinical settings: the indicated pH of dried vaginal secretions is low enough to be misdiagnosed as indicating vaginosis. Thus although the device disclosed in US Patent 5,897,834 is useful in a clinical setting where the health care professional applies the vaginal secretion to the device and observes the color change, if integrated in a patient useable device, such as a panty shield, the device gives abundant false positive results.

There is a need for an indicator system that can differentiate between a specific biological fluid of interest and an interfering biological fluid, such as, urine. Further there is a need for a device that can distinguish between normal vaginal secretions and those associated with amniotic fluid leakage or vaginosis. Further, a system in which false positive results are minimized, while reducing and the amount of time required to get the reliable result is also needed. Such a system is ideally useable by the patient to lead to greater peace of mind and to minimize unnecessary hospital visits. The characteristics of such an indicator system must not change due to long use or as a result of a wetting drying cycle and must distinguish between interfering biological fluids and minimize false positive readings. The present invention now overcomes these problems and satisfies these needs.

## SUMMARY OF THE INVENTION

The present invention, as defined in claim 1, overcomes the disadvantages of the prior art by the use of an indicator system integrated into various self useable products. Generally, the invention comprises an article that includes an absorbent material for absorbing a biological fluid secreted from a person and an indicator system that has at least one pH determining member and optionally, a reagent for reacting with the biological fluid to alter its pH so that the secretion can be distinguished. The indicator system is associated with an absorbent material such that the biological fluids contact the indicator system so that a reliable indication of the pH of that fluid can be obtained.

The article can be presented to the user in many forms. It is preferable, however, that the article is in the form of a swab, gauze, panty shield, hygienic napkin, a diaper or interlabial absorbent structure. Furthermore, any user, male or female, young or old, can use the article. The particular examples of the invention as presented herein are not intended to limit the scope of the invention, but simply to illustrate and represent the numerous potential forms in which the invention can be used.

Generally, the pH determining member can be anything that can indicate a pH of a fluid. Preferably the pH determining member records or indicates a pH change after coming in contact with the biological fluid and is resistant to change due to long use or wetting and drying cycles. Advantageously, the reagent used in the indicator system is one that reacts with amniotic fluid, a secretion associated with a bacterial, fungal, or yeast infection, or urine to change its pH.

Preferably, the article has a mounting means for positioning the absorbent body to receive the fluids secreted during the normal activity of the user, such mounting means being, for example, an adhesive strip or other attachment member.

A preferred embodiment is one in which the secretion-monitoring article has a substrate with a first pH indicator in a first area, a second pH indicator in a second area, and a reagent attached to the substrate in the second area, or alternatively a third area. The indicators are selected so that substantial color transitions occur at different pH values. A liquid contacting the substrate interacts with the indicators and the reagent. If the liquid has the pH of a fluid that is to be identified, at least part of the first area undergoes a substantial color change. The liquid may, however, be an interfering fluid with a pH that changes the color of the first indicator. Therefore, in one embodiment, the reagent is selected to react with the interfering fluid (for example), changing the pH of the liquid and consequently substantially changing the color of at least part of the second area. The presence of the second pH indicator acts as a guarantee against false positive results by allowing a colorimetric differentiation of two fluids with a similar pH.

According to another feature of the present invention, the first pH indicator changes color at a substantially lower pH than does the second pH indicator. Usually, the first area is distinct from the second area and the shape of the area can vary and be any geometrical shape, number, letter, icon, word or a combination thereof.

Another non-limiting embodiment of the invention is a secretion-monitoring article wherein the indicator system has at least one pH determining member having a chemical composition that reacts with biological fluids that contain protonated amine cations differently than bodily fluids that do not contain protonated amine cations. Typically, the indicator system is associated with the absorbent material such that the biological fluids contact the indicator system while being worn.

Advantageously, the secretion-monitoring article can be used for the identification of infected urine. In this embodiment the article comprises a body that includes an absorbent material for absorbing urine from a person and an indicator system that has at least one indicator having a chemical composition that reacts with normal urine differently than infected urine. The indicator in this embodiment changes color when contacted by urine, but if the urine is infected the color change of the indicator is nonreversible.

An additional embodiment is one in which the secretion-monitoring article is designed specifically for the identification of bacterially infected vaginal secretions. In this embodiment the article comprises a body that includes an absorbent material for absorbing vaginal secretion and an indicator system that has at least one indicator having a chemical composition that reacts with normal or candida vaginal secretions differently than bacterially infected vaginal secretions, wherein the indicator changes color when contacted by bacterially infected vaginal secretion having a pH level above 5 and a concentration of amines higher than normal or candida vaginal secretions.

Another preferred embodiment of the invention comprises an article that includes a pH determining member with a composition that reacts differently to urine than other biological fluids, such as amniotic fluid. The composition of the pH determining member is able to react differently due to certain chemicals that are present in substantial amounts only in urine, and not in the other biological fluids to be identified. The substrate containing the pH determining member is retained in the vicinity of a vaginal area of the person for an extended period to absorb the fluids. After which the article is removed and observed to determine the health condition of the person from which the biological fluid was collected.

There is also included a method for providing an indication of the health condition of a person by providing a substrate to which are attached a first pH indicator in a first area, a second pH indicator in a second area, and a reagent attached to the substrate in the second area, where the color transitions of each of the two indicators occur at a substantially dissimilar pH. A liquid, such as a biological fluid is applied to the substrate and the first and second area is inspected for a change in color indicative of the health condition of the person. According to a feature of the present invention, the substrate is retained in the vicinity of a vaginal area of the person for an extended period of time such as minutes, hours or even longer, to absorb secreted fluids.

According to a further feature of the present invention, the first pH indicator is configured to substantially change color upon contact with amniotic fluid and the second pH indicator is configured to substantially change color upon contact with urine reacting with the reagent. A preferred the reagent is urease. Alternatively, the first pH indicator is configured to substantially change color upon contact with vaginal secretions associated with vaginosis and the second pH indicator is configured to substantially change color upon contact with urine reacting with the reagent.

Also disclosed is a method of attaching an indicator to a substrate. The substrate can be made of many materials, for example, polypropylene, paper or cotton, polyester membranes and can be of many structures including of a membrane, fabric, mesh, gauze, thread, fiber and a sheet. A mixture of pre-formed polymer (such as a cellulose), a plasticizer, a wetting agent, an ion-balance reagent and an indicator (alone or with a reagent such as urease) is prepared. In some cases it is preferable to add a solvent to the mixture. The mixture is applied to a substrate for example by dipping the substrate in the mixture or by spraying or spreading the mixture onto the substrate. The substrate with the applied mixture is allowed to dry. When dry, the indicator is bound to the substrate with the help of the polymer. This method is exceptionally useful when the indicators have a substantially negatively charged functional group such as an acetate or a sulfonate.

There is also provided an additional method of making a diagnostic article comprising of the steps of attaching an indicator to a substrate, especially a neutral substrate, by applying a surfactant solution to the substrate and letting it dry, preferably under vacuum, then once the surfactant is dry, an indicator solution or a solution with a reagent is applied to the substrate and allowed to dry, preferably under vacuum, wherein the indicator to be attached to the substrate preferably is a substantially negatively charged functional group with a cationic surfactant is preferably used; and placing the indicator in association with an absorbent body.


## BRIEF DESCRIPTION OF THE DRAWINGS

The invention is herein described, by way of example only, with reference to the accompanying drawings.

FIGS. 1A-C are schematic top views of an embodiment of the secretion-monitoring article of the present invention with magnification of details of indicators applied to the substrate.

FIG. 2 is a schematic top view of a different embodiment of the secretion-monitoring article of the present invention.

FIG. 3 is a schematic perspective view of a different embodiment of the secretion-monitoring article of the present invention with a microporous membrane.

FIG. 4 A-C are schematic top view of an embodiment of the secretion-monitoring article with two pH indicators.

FIG. 5 A-B are schematic top view of an embodiment of the secretion-monitoring article with one pH indicator device that can distinguish between urine and other body fluids, such as amniotic fluid.

## DETAILED DESCRIPTION OF THE PREFERRED EMBODIMENTS

Before turning to details of the present invention, it should be appreciated that the present invention provides secretion-monitoring article and a method for use that allows an untrained user to monitor secreted biological fluids with confidence. The present invention allows for the identification of a specific biological fluid even when there is a possibility for the presence of an interfering biological fluid with a similar pH.

The present invention is an improvement over the prior art, providing a secretion monitoring article that is more reliable and convenient for the user.

In one embodiment of the invention, the secretion-monitoring article comprises a body that includes an absorbent material for absorbing a biological fluid secreted from a person and an indicator system comprising at least one pH determining member and a reagent for reacting with the biological fluid to alter its pH so that the secretion can be distinguished, wherein the indicator system is associated with the absorbent material such that the biological fluids contact the indicator system.

In a second embodiment of the invention, the secretion-monitoring article comprises a body that includes an absorbent material for absorbing a biological fluid secreted from a person and an indicator system comprising a pH determining member that consist of a special composition that reacts with biological fluid differently. One example of such a pH determining member has a special composition that reacts with fluids containing protonated amine cations, such as urine, in a different way than it reacts to other biological fluids that have a low concentration of protonated amine cations, such as amniotic fluid.

In yet another embodiment of the invention, the secretion-monitoring article comprises a body that includes an absorbent material for absorbing a biological fluid secreted from a person and an indicator system comprising a pH determining member that consist of a special composition that reacts with normal urine differently than infected or protein containing urine. In one non-limiting example, the indicator reacts with normal urine (pH 5-8), which changes the color from yellow to green or turquoise. During the drying process the color change of the indicator that has been contacted with normal urine fades as it dries and becomes yellow again. In contrast, when the indicator is contacted by infected or protein containing urine the indicator strip changes color from yellow to green or turquoise and does not fade when dried. Advantageously, this embodiment is well suited for all types of use, for example in pediatrics, geriatrics, and gynecology, and could be presented to the user in many forms, preferably as a diaper or a panty liner.

The secretion-monitoring article can be implemented using many devices and methods. In a preferred embodiment, the article of the present invention is implemented in a manner that can be easily used by non-skilled personnel, specifically a user. The body of the secretion-monitoring article of the present invention comprising the absorbent material can be supplied to the user, for example, in the form of a pad, gauze, a swab, a fiber ball, but most preferably, as a sanitary napkin, diaper, panty shield, and interlabial structure. Details of manufacture of these are well known to one skilled and have been fully described in the prior art, for example US Patents 5,217,444, 5,897,834, and 6,149,590.

Furthermore, any user, male or female, young or old, can use the article in a variety of forms. The particular examples of the invention as presented herein are not intended to limit the scope of the invention, but simply to illustrate and represent the numerous potential forms in which the invention can be used.

In one embodiment of the invention, an indicator system made up of a pH determining member and a reagent to be included in the body of the secretion-monitoring article is provided. In another embodiment, an indicator system is made up of pH determining member that reacts differently to different body fluids.

The pH determining member of the indication system can be any pH determining device, for example as a color changing indicator (e.g., litmus paper) or a mobile pH probe. It is preferable, however, that the pH determining member be a color changing indicator, such as a pH determining member made from the pH indicator mixture described herein below and/or using the method of attaching the mixture to a substrate. As will be discussed in more detail below, more than one pH determining member can be part of the indicator system. The pH determining members should be capable of determining substantially different pH ranges or capable of reacting differently to different biological fluids to produce a different color change.

In one embodiment the indicator system comprises a reagent. The reagent is used to distinguish the pH of the biological fluid being monitored from other biological fluids that might interfere with the results and possible give a "false positive" result, with unwarranted stress and expense to the user. The reagent can be chosen based on the biological fluid to be monitored and the type of biological fluids that might interfere with the accurate monitoring of this fluid. The reagent of the indicator system of the present invention is chosen so as to yield reaction products that substantially change the pH of a tested secretion when the tested secretion is either the fluid to be identified or is the interfering fluid, or both. If the reagent is chosen so as to react with both fluids, the pH change resulting from reaction with the fluid to be identified should be different from the pH change resulting from the reaction with the interfering fluid.

In one preferred non-limiting embodiment of the present invention, when either amniotic fluid is to be identified or vaginosis is to be diagnosed, urease (CAS 9002-13-5) is chosen as the reagent. If urine is present, the urine reacts with the urease, releasing ammonia into the tested secretion raising its pH to well above the pH of either amniotic fluid or vaginosis related secretions.

The reagent can also be chosen to react only with the fluid to be identified, change the pH sufficiently to distinguish it from any possible interfering fluid. When the reagent is chosen to only react with the fluid to be identified, it is preferable that the reagent reacts with amniotic fluid or a secretion associated with a bacterial, fungal, or yeast infection and that the pH is sufficiently changed so as to distinguish the fluid from other possible interfering fluids.

In one embodiment of the invention, the indicator system has only one pH indicator and the reagent is selected to react with the fluid to be identified so that the pH is changed substantial so that the presence of the fluid can easily be identified. In this embodiment of the invention, the pH indicator is selected to indicate the pH of the fluid to be identified after it has reacted with the reagent.

In a preferred embodiments of the secretion-monitoring article, a means for mounting the article to facilitate the collection of the secreted biological fluid is included. An example of a mounting means that is well known in the art is an adhesive strips associated with the article. In a preferred embodiment the article has one or more adhesive strips. The user removes the release tape to expose the adhesive strip of the article and places the article in the crotch portion of their undergarment. This prevents the article from moving out of position during regular use. Types of adhesive compounds that can be used are well know in the art.

## Examples of the secretion-monitoring articles

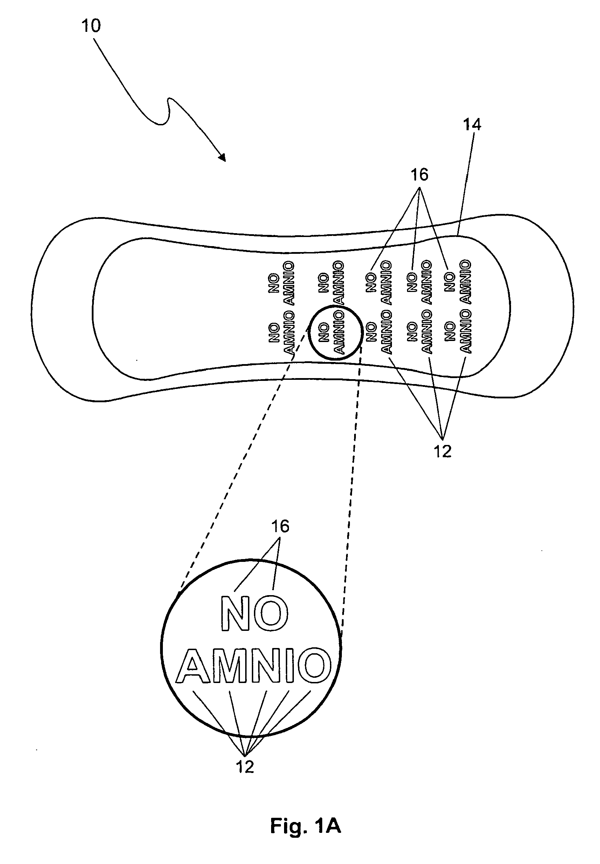

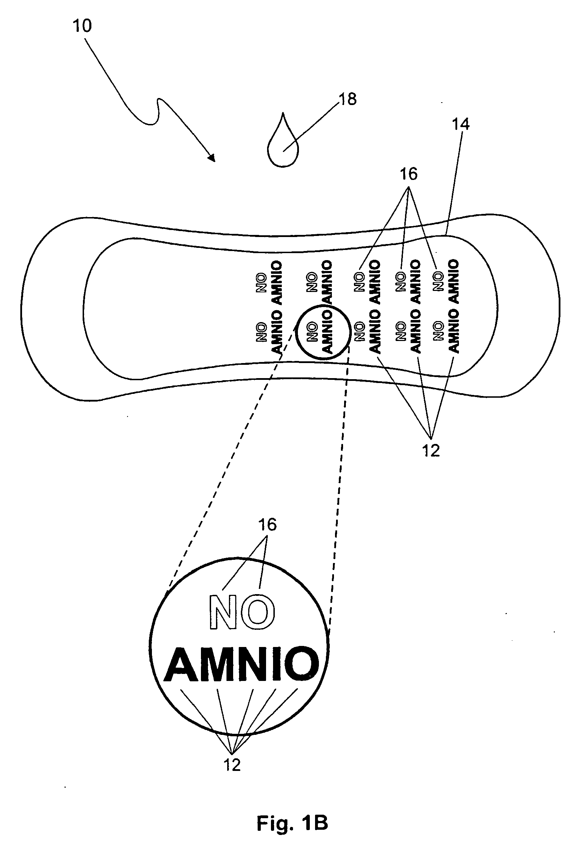

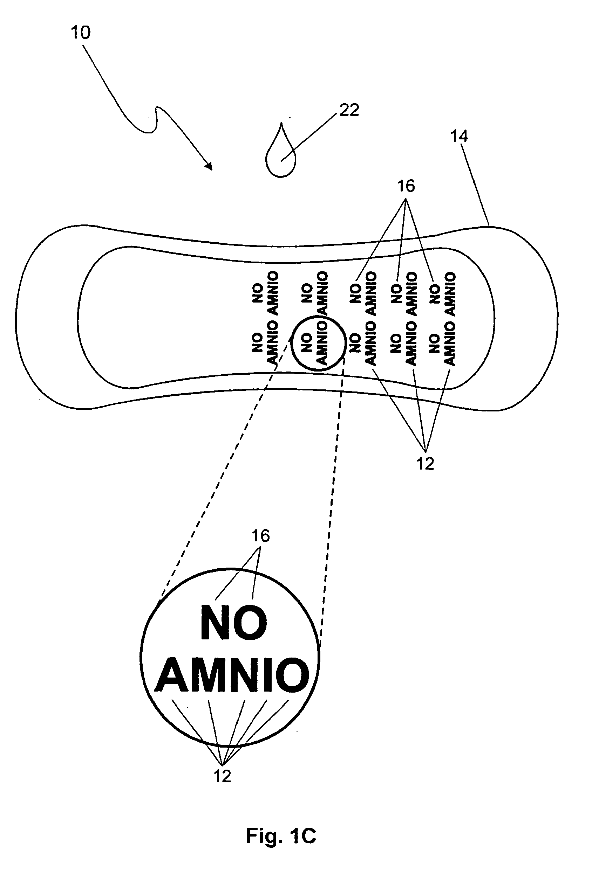

The present invention will be exemplified by embodiments of the secretion-monitoring article of the present invention in the form of a panty shield in Figures 1-5. The article can be configured to identify amniotic fluid and secretions associated with bacterial, fungal, or yeast infection, such as infected urine or vaginal secretions. Furthermore, the article is designed to minimize false positive readings associated with interfering biological fluids.

A preferred embodiment of the invention is one in which the indicator system comprises a first indicator, a second indicator and a reagent. The first indicator of the system of the present invention is chosen so as to identify a first pH. The first pH corresponds to the pH of the fluid that is to be identified. Further, the first pH may also be that of an interfering fluid. When amniotic fluid is to be identified, a first indicator is chosen to indicate that a tested vaginal secretion has a pH of amniotic fluid. Due to the similar pH of urine and amniotic fluid, such a first indicator will also change color when exposed to urine. When vaginosis is to be to diagnosed, a first indicator is chosen to indicate a pH typical of secretions of vaginosis and consequently also of urine. In Figure 1A, a first indicator is applied at first area 12 on a substrate 14 integrated in a panty shield 10. First area 12 may be arranged as patterns, letters, words or icons, as described in US Patent 5,897,834. In Figures 1A-C, first indicator is nitrazine yellow, which is yellow at a pH below 7 and blue violet at a pH above 7.

The reagent of the indicator system of the present invention is chosen so as to yield reaction products that substantially change the pH of a tested secretion as described in detail above.

The second pH indicator of the system of the present invention is chosen so that it indicates the change of pH as a result of the reaction with the reagent. For example, second indicator in Figures 1A-C is m-cresol purple. m-cresol purple is yellow at a pH of below 7.5 and is violet at a pH above 8.0. The second indicator and the reagent are applied at second area 16 on substrate 14, distinct from the first area 12 on substrate 14, Figure 1A.

In Figure 1B, amniotic fluid 18 comes in contact with panty shield 10. Amniotic fluid 18 makes contact with first area 12 and second area 16. As the pH of amniotic fluid 18 is between 7.0 and 7.5, the nitrazine yellow present at first area 12 become blue violet, spelling out the word "AMNIO". It is clear to one skilled in the art that if a small amount of fluid is applied to panty shield 20, it is possible that only part of first area 12 will change color. The m-cresol purple present at second area 16 remains yellow.

When the user of panty shield 20 in Figure 1B examines panty shield 10, she reads the word "AMNIO" and can go to a health-care professional who can take action corresponding to a high degree of certainty of amniotic fluid secretion.

In Figure 1C, urine 22 comes in contact with panty shield 10. Urine 22 makes contact with first area 12 and second area 16. As the pH of urine 22 is 7.2, the nitrazine yellow present at first area 12 becomes blue violet, spelling out the word "AMNIO". Urine 20 reacts with urease present at second area 16, releasing ammonia. The ammonia increases the pH of the liquid present in second area 16 to pH 9. As a result of the high pH, m-cresol purple present at second area 16 becomes violet, spelling out the word "NO".

| TABLE 1 | | | |
|---|---|---|---|
| Indicator | aqueous pH transition range | color change | CAS |
| 1. Cresol Red | 7.2-8.8 | yellow to reddish purple | 1733-12-6 |
| 2. Alizarin | 5.5-6.8 | yellow to violet | 72-48-0 |
| 3. Bromcresol Purple | 5.2-6.8 | yellow to purple | 115-40-2 |
| 4. Chlorophenol Red | 5.2-8.8 | yellow to red | 4430-20-0 |
| 5. Nitrazine Yellow | 6.0-7.2 | yellow to bright blue | 5423-07-4 |
| 6. Bromthymol Blue | 6.0-7.6 | yellow to blue | 34722-90-2 |
| 7. Bromoxylenol Blue | 6.0-7.6 | yellow to blue | 40070-59-5 |
| 8. Neutral Red | 6.8-8.0 | red to yellow | 553-24-9 |
| 9. Phenol Red | 6.8-8.2 | yellow to red | 34487-61-1 |
| 10. Thymol Blue | 8.0-9.2 | yellow to blue | 81012-93-3 |
| 11. Xylenol Blue | 8.0-9.6 | yellow to blue | 125-31-5 |
| 12. m-Cresol purple | 7.4-9.0 | yellow to purple | 2303-01-7 |

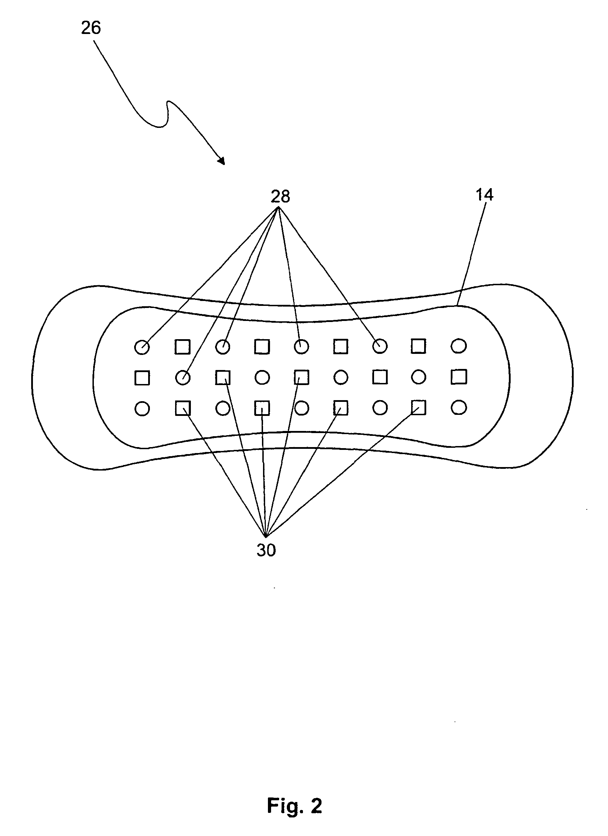

When the user of panty shield 24 in Figure 1C examines panty shield 10, she reads the words "NO AMNIO". The user who became agitated at the unexpected loss of fluid is immediately calmed and is relieved of the necessity of a stressful visit to a health-care professional. It is clear to one skilled in the art that arranging first area 12 and second area 16 so as to spell out words is not necessary, and in alternative embodiments of the present invention first area 12 and second area 16 may have any shape. For example, in Figure 2, a panty shield 26 configured in accordance with the present invention is depicted where each one of first area 28 is of substantially circular shape and each one of second area 30 is substantially square shaped.

When used in a medical setting, it is imperative that there be substantially no leaching of indicator system components from the substrate to which the indicator system is attached. The attachment of indicators to a substrate is well within the ability of one skilled in the art. One family of chemical compounds that are suitable for use as a first indicator and a second indicator of the preferred embodiment of the present invention without leaching are indicators with negative functional groups. Suitable indicators include nitrazine yellow, thymol blue, bromthymol blue, xylenol blue, bromoxylenol blue, phenol red, m-cresol purple, chlorophenol red, bromcresol purple, alizarin, neutral red, and cresol red, see Table 1. A list of other suitable indicators can be found, for example, in US Patent 5,897,834. It is clear to one skilled in the art that the indicators specifically mentioned herein are just examples and any suitable indicators may be used. Further, there may be instances where the first indicator and/or the second indicator are made up of a combination of individual indicators.

Another non-limiting embodiment of the indicator system of the present invention is a secretion-monitoring article for the identification of vaginal infections such as bacterial vaginosis (BV). According to the present invention, an indicator system is made with a first indicator that indicates the presence of a fluid with a pH of around 4.7 to 7.0. The first indicator can be chosen, for example, from one or more of the group including nitrazine yellow, bromthymol blue and bromoxylenol blue. As can be seen in Table 1, these three indicators typically exhibit a bluish color when exposed to a fluid with a pH above 7.0. The second indicator can be chosen, for example, from the group including phenol red, thymol blue, xylenol blue and m-cresol purple. As can be seen in Table 1, upon exposure to a fluid with a pH above 8.0 these four indicators become red, blue, violet and violet, respectively. The reagent added to the second embodiment of the secretion-monitoring article of the present invention is, for example, urease.

As discussed hereinabove, urine of a healthy patient has a pH between 5.0 and 8.0. A patient having BV also has vaginal secretions with a pH between 4.7 and 6.5. If the liquid examined in the second embodiment of the secretion-monitoring article of the present invention is associated with BV, the first indicator changes color whereas the second indicator remains yellow. If the liquid examined contains urine, the first indicator changes color. Further, the urease reacts with the urine, producing ammonia, raising the pH of the fluid, and consequently causing the second indicator to change color.

The following examples set forth preferable embodiments of the present invention.

**Example 1:** Reducing erroneous readings of color-changing devices that give an indication of elevated pH in the vaginal secretion

The following example discloses the solution to produce an indicator that needs no color-table or scale to read results, that shows the user a stable indication for a few days, and that does not leach even when in contact with liquids for any practical length of time. For the non-invasive continuous monitoring version, the invention discloses a solution to avoid false positive readings due to urine contamination.

The device is a sticker or a pantyliner that contains two different indicator strips, embedded between layers of one-way absorbent tissues. The two indicators have a color transition-point at different pH levels. The color-reactions of the two indicators also have different reversibility in vaginal secretion Vs urine.

The first indicator strip changes color to stable blue, when sensing elevated pH in vaginal secretions (pH strip). The pH strip contains the pH indicator-Nitrazine-yellow, which has a pKa of 6.6 in aqueous solution, and with the innovative specific composition, changes the color when the vaginal secretion has a pH level of 5.0 or higher (the same innovative specific composition produces indicators for various pH levels, by using other negatively-charged members of the Ionizable phenol group).

The second strip is a control strip to detect urine (urine strip). The urine strip contains urease, an enzyme which hydrolyzes urea to ammonia, and a pH indicator m-Cresol purple which has a pKa of 8.2 in aqueous solution. When the strip is in contact with urine, the hydrolyzed ammonia raises the pH of the medium and the color of the m-Cresol purple changes from yellow to dark gray/green.

In a case where vaginal secretion with elevated pH (5.0-7.0) will reach the strips only the pH strip will change color and the change will remain stable for a few days.

**Method of Preparation**

1) pH strip:

**Step 1:** To a 10 ml of Acetone add 150 mg Cellulose acetate, 107 $\mu$l Dibutylphthalate, 23$\mu$l Aliquat, 150 $\mu$l 2-Ethoxy ethanol and 2.4 mg Nitrazine yellow dissolved in 150 $\mu$l DDW.
**Step 2:** Stir the mixture for few minutes to complete dissolving.
**Step 3:** Coat a polyester monofilament screening fabric with the polymer solution (coating other materials unsensitive to acetone will produce various devices for various using instructions, with the same features).

2) Urine strip:

**First layer-step 1:** To a 4.15 mL DDW add 45 mg PVP, 0.325 mL Urease/glycerol solution.
**First layer-step 2:** Coat a polyester monofilament screening fabric with the polymer solution.
**First layer-step 3:** The coated strips are dried-out over night at room temperature.
**Second layer-step 1:** To a 10 ml of THF add 150 mg Cellulose acetate, 107 $\mu$l Dibutylphthalate, 23$\mu$l Aliquat, 150 $\mu$l 2-Ethoxy ethanol and 1.2 mg m-Cresol purple dissolved in 120 $\mu$l 1-Propanol.
**Second layer-step 2:** Stir the mixture for few minutes to complete dissolving.
**Second layer-step 3:** Coat the strip with the second polymer solution.
**Second layer-step 4:** After drying over night the wash the strip in a saline solution.

The device can be in the form of a swab with a tip produced in the same way as mentioned above, under the header: pH strip. The tip may be prepared by using a short strip, rolled on the stick of the swab, or by coating the tip of an integrated swab (implementing step 3), where the tip consists of any screening fabric.

**EXAMPLE 2:** A device able to distinguish accurately between an amniotic fluid leak or an elevated pH vaginal discharged secretion and wetness caused by urine incontinence

Due to the severe consequences of amniotic fluid leakage, pregnant women undergo heavy stress and tend to seek for a health care provider upon any wet sensation in the area of the vagina. The common ways to checks for the presence of amniotic fluid are by examining the pH of vaginal secretions with pH indicators such as Nitrazine indicators, running the Fern-test or by visually identifying the source of the leakage.

Amniotic fluid has a pH level that varies between 6-8 and can be identified by a purple-blue color of a Nitrazine indicator. Since urine, has a pH level that varies between 5.0-8.0, measuring pH levels as a sole criterion can mislead to erroneous decisions. As the other two ways can be performed only in clinics and hospitals, and by trained staff, there is no practical solution for home monitoring. In some situation, after amniocentesis tests and other occasions such as hi-risk pregnancies, there is a possibility of small amniotic leaks that can be detected only by continuous monitoring.

Current solutions and earlier inventions fail to serve as a home-use continuous monitoring device, as they leach in fluids, the color change is unstable, and the overlap between amniotic fluid pH level and the urine pH level misleads the users in as 30% of the cases.

The overlap of pH levels, between amniotic fluids and urine is also a great disadvantage for physicians treating patients with wet sensations. Providing pregnant women with a home-use continuous monitoring device, that distinguishes amniotic fluid leakage from urine incontinence with no false alarms, enabling the result reading at personal timing and discretion, and detects any small amniotic leak instantaneously, can on one hand help bring the user in-time to hospital when needed, and on the other hand avoid unnecessary hospitalization and concomitant patient stress.

Providing physicians with a reliable clinic instantaneous detecting article, that distinguishes amniotic fluid leakage from urine incontinence with no false alarms, can serve them by far better than available solutions today.

The article can be a sticker or a pantyliner with an embedded indicator strip. The strip contains the pH indicator-Nitrazine-yellow which has a pKa of 6.6 in aqueous solution.

Reaction of the indicator with amniotic fluid (pH 6-8) changes the color from yellow to stable dark blue. Reaction of the indicator with urine (pH 5-8) changes the color to fading green or fading turquoise. Urine with lower pH 5-5.5 doesn't change the indicator color.

The difference between the color reaction of the indicator with amniotic fluid and with urine consists of two parameters: the chemical composition of the fluids and the indicator's polymer chemical structure.

The following two equations demonstrates the different reactions

$$\text{Equation 1:} \quad R\!-\!OH + X^- \ + \ NR_4^+Cl^- \ \rightleftharpoons \ R\!-\!O^-\,NR_4^+ + H_2O + Cl^-$$

$$\text{Yellow} \qquad\qquad \text{Ion balance} \qquad\qquad \text{Ion pair}$$

$$\text{Reagent} \qquad\qquad\qquad \textbf{Blue}$$

$$\text{Equation 2:} \quad R\!-\!OH + X^- \ + \ NH_4^+Cl^- \ \rightleftharpoons \ R\!-\!O^-\,NH_4^+ + H_2O + Cl^-$$

$$\text{Yellow} \qquad\qquad \text{Ammonium} \qquad\qquad \text{Ion pair}$$

$$\textbf{Turquoise}$$

KEY:

$X^-$ = Base.
$NR_4^+$ = Ion balance reagent
$R\text{-}O^-\,NR_4^+$ =- phenolate - ion-balance reagent

The ratio of ion-balance reagent versus indicator in the polymer matrix controls the transition point of the color and the color stability while drying. In the drying process the ion pair phenolate (the active site of the indicator) - ion-balance reagent is stable, which cause a stable performance of the color (equation 1 - the relative concentration of the component doesn't change). In a different case where the concentration of the ion-balance reagent in the polymer is higher, the color of the indicator is getting dark while drying. The color darkening while drying is due to continuance deprotonation of the indicator's phenol by the basic excess of the ion balance reagent (equation 1- while drying the base concentration is getting high and the equilibrium turned to the right). The optimum molar ratio of ion-balance reagent to indicator is 10:1.

Ammonium ions in solution react like the ion-balance reagent and compete with the phenolate active site. While drying the ion pair phenolate - Ammonium hydrolyzed spontaneously to give the protonated yellow phenol (equation 2) while the phenolate - ion-balance reagent pair is stable (equation 1).

In a case where the medium contain ammonium ion the color changes govern by the relative concentration of the ion balance reagent in the polymer and the ammonium ion in the medium.

For example: in 100mM buffer solution that contains 25mM ammonium ion the concentration of the ammonium is in two orders of magnitude higher then the ion balance reagent in the polymer. These differences govern the turquoise color in solution and the fading color on drying.

Urine contains ammonium ions in concentration of 30 - 50 mM; amniotic fluid doesn't contain any substantial amount of ammonium ions, thus causing no fading influence as urine does.

Method of Preparation:

**Step 1:** To a 10 ml of Acetone add 150 mg Cellulose acetate, 107 $\mu$l Dibutylphthalate, 23$\mu$l Aliquat, 150 $\mu$l 2-Ethoxy ethanol and 2.4 mg Nitrazine yellow dissolved in 150 $\mu$l DDW.
**Step 2:** Stir the mixture for few minutes to complete dissolving.
**Step 3:** Coat a polyester monofilament screening fabric with the polymer solution to give the desired product.

The device can be a swab with a tip produced in the same way as mentioned above, under the header: pH strip. The tip may be prepared by using a short strip, rolled on the stick of the swab, or by coating the tip of an integrated swab (implementing step 3), where the tip consists of any screening fabric.

**EXAMPLE 3:** A device able to distinguish accurately between normal urine and infected urine

The reoccurrence of urinary tract infections in certain patients present the need to quickly and easily diagnose whether the patient has another urinary tract infection. Presently, to determine if a patient has a urinary tract infection they must make an appointment to visit a doctor. Furthermore, if the patient is susceptible to the reoccurrence of urinary tract infections they must make periodic visits to the doctor's office to ensure that the infection has not reoccurred. Having a device that would allow the user to determine if they had a urinary tract infection again would minimize stress and time consumed by visits to the doctors office and result in quicker diagnosis of the infection, resulting in a reduction in pain suffered by the patient and a more timely treatment of the infection.

The article in this example is a diaper or a panty liner with an indicator that can distinguish between normal urine and infected urine. The user wears the article so that urine can come in contact with the article. The reaction of the indicator with urine (pH 5-8) changes the color from yellow to green or turquoise. The drying process of the indicator strip at room temperature is short (5 minutes). When normal urine comes in contact with the indicator strip the color changes fade while drying. The color change is completely reversible and the strip becomes yellow again. On the other hand when infected urine comes in contact with the indicator strip the color changes to green or turquoise and stay constant while drying.

The reversibility of the color changes depends on two different factors of the environment:

1. Chemical environment:

   a. The pH level of the fluid - pH level higher than the pKa gives a stable color change.
   b. Buffer capacity of the solution - in weak buffer solution the indicator plays the rule of weak acid and the reaction is reversible, while in strong buffer solution the color change is stable.
   c. Ammonium salts content in the solution - explained extensively in EXAMPLE 2.

2. Biological environment:

   a. Protein presence in urine gives a stable color change and the reaction is not reversible.

Infected urine provides a stable color change to the indicator, which color change is not reversible. Furthermore, bacteria presence in vaginal secretion fluid also gives a stable color change so that the color change is not reversible.

**EXAMPLE 4:** A device able to distinguish between normal or candida vaginal secretions and bacterially infected vaginal secretions

In this embodiment, Applicants have been able to provide information regarding two of the Aamsel criteria with a single indicator.

The article in this example is typically a panty liner with an indicator that can distinguish between normal or candida vaginal secretions and bacterially infected vaginal secretions. The user wears the article so that urine can come in contact with the article. The indicator is designed to change color when the secretion has a pH above 5 and high concentrations amines. Advantageously, only one indicator is necessary to distinguish detect a bacterially infected secretion, being able to determine two of the Aamsel criteria, pH and amine concentration. Other indicators may be present in the article, but are not necessary to properly determine whether the user has vaginosis.

The user wears the article and if the user has bacterial vaginosis, the bacterially infected vaginal secretion will cause the indicator to change color due to the high concentration of amines and pH above 5. Whereas, a normal or candida vaginal secretion will not cause the indicator to change colors since they do not have both a pH above 5 and a high concentration of amines. In this context a high concentration of amines refers to a concentration above about 0.4mM to 0.5mM.

## Improved methods for attaching indicators to a substrate

Details and variations concerning the method of manufacture of a secretion-monitoring article for implementing the indicator system of the present invention or applying the method of the present invention are well described in the prior art.

As described hereinabove, US Patent 5,897,834 describes a solid pre-formed polymer to which quaternary ammonium groups are covalently bound. Negatively charged indicators are non-covalently bound to the polymer. The non-covalent bonds are strong enough so that the attached indicators do not leach out in an aqueous solution. In addition, the indicators bound to the polymer have a sharpened pH color transition, allowing an accurate determination of the pH of the applied fluid. The polymer can be applied to various substrates. However, indicators bound to these polymers are less useful in non-clinical settings as the indicated pH of vaginal secretions after drying is lower than that of fresh vaginal secretions, leading to a false positive results.

It is disclosed a method suitable for attaching indicators to a substrate so that the indicators do not leach out in an aqueous fluid. Especially suitable indicators are those with a negatively charged group, such as those listed in Table I or, for example, in US Patent 5,897,834. The disclosed polymer is exceptionally suited for attaching the indicator system of the present invention to a substrate. Further, experiments show that unlike other methods and polymers known in the art, changes in color of indicator attached according to the disclosed methods are fast. The color is retained over a long period of time and even when the applied liquid dries. Repeated cycles of drying and wetting also do not change the color. Thus, in practical terms, there is time for a user to get to a health care professional without the color of the indicator changing.

## Application of indicator to a substrate

In a first method of attaching an indicator to a substrate , an indicator is mixed with a preformed polymer in a suitable solution and then applied to a substrate.

In more detail, a polymer solution is prepared containing dry pre-formed polymer, plasticizer, a wetting agent, an ion-balance reagent, a solvent and an indicator. When practicing the method, a reagent as described is also added.

The preformed polymer can be selected from various preformed polymers, although cellulose polymers such as nitrocellulose, cellulose acetate or ethyl cellulose are preferred. The preformed polymer makes up 20% to 50% of the weight of the solution. Preferred is that the polymer makes up 25% to 45% of the solution, more preferred is that the polymer makes up 30% to 43% of the solution, and most preferred is that the polymer makes up 36% to 39% by weight of the solution. As is clear to one skilled in the art, it is also possible to use a combination of suitable preformed polymers when making one polymer solution.

Although any suitable plasticizer can be used, bis-(2-butoxyethyl) adipate (BBPA, CAS 141-18-4), bis-(2-ethylhexyl) sebacate (DOS, CAS 122-62-3), diethyl phthalate (DEP, CAS 84-66-2) or dibutyl phthalate (DBP, CAS 84-74-2) are preferred. The plasticizer makes up 15% to 40% of the weight of the solution. Preferred is that the plasticizer makes up 20% to 35% of the solution, more preferred is that the plasticizer makes up 25% to 31% of the solution, and most preferred is that the plasticizer makes up 27% to 29% by weight of the solution. As is clear to one skilled in the art, it is also possible to use a combination of suitable plasticizers when making one polymer solution.

Although any suitable wetting agent can be used, triethylene glycol, ethylene glycol, sorbitol or 2-ethoxy ethanol are preferred. The wetting agent makes up 15% to 45% of the weight of the solution. Preferred is that the wetting agent makes up 21% to 40% of the solution, more preferred is that the wetting agent makes up 26% to 34% of the solution, and most preferred is that the wetting agent makes up 29% to 31 % by weight of the solution. As is clear to one skilled in the art. it is also possible to use a combination of suitable wetting agents when making one polymer solution.

Although any suitable ion-balance reagent can be used, tricaprylylmethyl ammonium chloride (Aliquat 336, CAS 5137-55-3), tridodecylmethyl ammonium chloride (TDMAC. CAS 7173-54-8) or cetyltimethyl ammonium chloride (CTAC, CAS 112-02-7) are preferred. The ion-balance reagent makes up 0.1% to 10% of the weight of the solution. Preferred is that the ion-balance reagent makes up 1 % to 8% of the solution, more preferred is that the ion-balance reagent makes up 3% to 7% of the solution, and most preferred is that the ion-balance reagent makes up 4% to 6% by weight of the solution. As is clear to one skilled in the art, it is also possible to use a combination of suitable ion-balance reagents when making one polymer solution.

The components of the solution are added so that the sum of weights of pre-formed polymer, plasticizer, wetting agent and ion-balance reagent is equal to 100%.

The desired indicator is added to the solution. Although any suitable indicator can be used, it is preferred that the indicator molecules have a negatively charged functional group such as acetate or sulfonate. Most preferably, the indicators used, separately or in combination, are chosen from amongst indicators listed in Table 1 and in US Patent 5,897,834. The total amount of indicator added is 0.05% to 5% of the weight of the polymer solution as described above. Preferred is that the indicator is 0.05% to 3% of the polymer solution, more preferred is that the indicator is 0.1% to 1% of the polymer solution, and most preferred is that the indicator is 0.2% to 0.4% of weight of the polymer solution.

When it is desired to add a reagent in preparation of the indicator system of the present invention, reagent is added to the polymer solution. For example, when urease is used any suitable amount of urease can be added although it is preferred that the concentration of urease is about 10 units for each 0.01% - 0.1% of indicator added to the polymer solution.

Further, an amount of solvent is added that is suitable for making any easily applied solution / indicator mixture. Typically, 150 mg of polymer solution is dissolved in between 1 ml and 30 ml of solvent, preferably between 5 ml and 15 ml solvent. Although any suitable solvent or mixture of solvents may be used, preferred are ethyl acetate or substantially volatile ethers such as diethyl ether, isopropyl ether, t-butyl methyl methyl ether or tetrahydrofuran

Once the mixture is ready, it is applied by suitable means to the substrate. Application can be done, for example, by spraying or spreading the mixture on the substrate, or by dipping the substrate in the mixture. The substrate can be of many suitable materials known in the art such as polyester membranes, polypropylene membranes, cellulose membranes, paper, cotton or linen. The structure of the substrate may be, for example, a fiber, a mesh, gauze, a fabric or a membrane. The solvent of the mixture is allowed to evaporate. Once the mixture dries onto the substrate, the substrate is integrated into whatever secretion-monitoring article is desired, such as a panty shield.

As is clear to one skilled in the art that when the indicator system of the present invention is implemented, a first mixture with a first indicator is made, and a second mixture with a second indicator and a reagent is made, both mixtures as described hereinabove. Each of the two mixtures is applied to area on the substrate, as described hereinabove. Preferably the area of application of the first mixture is substantially distinct from the area of application of the second mixture.

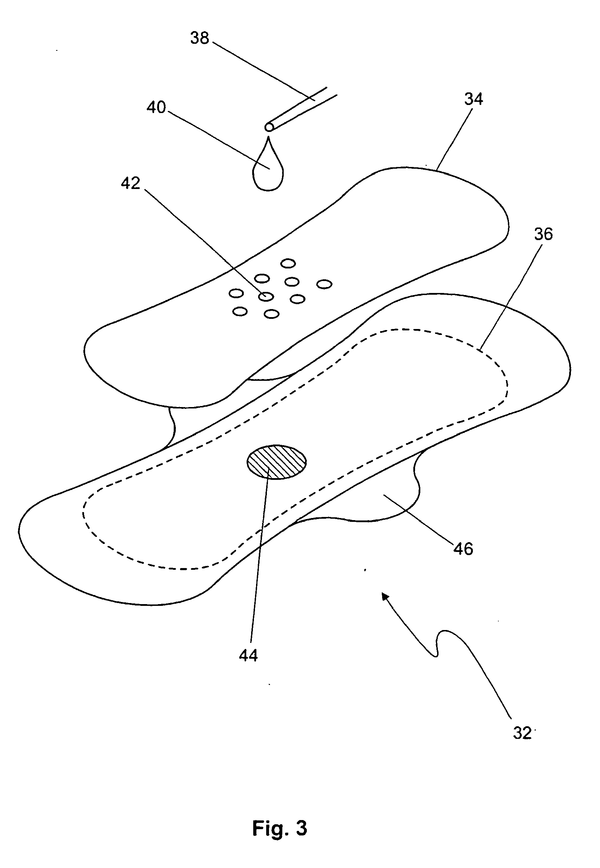

In certain applications, the liquid to be tested may contain biological polymers such as proteins or fats. For example, amniotic fluid and urine often contain proteins. The biological polymers may plug up the pores in the substrate reducing the effectivity of the testing method. This can be exceptionally significant in panty shield applications such as panty shield 32 depicted in Figure 3. In such a case, it is preferable to interpose a microporous membrane 34, such as a dialysis membrane (e.g., cellulose membrane, catalog nr. D-9402, Sigma-Aldrich, St. Louis MO), between indicator substrate 36 and a source 38 of secretion 40. Large-sized materials 42 in secretion 40 cannot penetrate microporous membrane 34 whereas fluid component 44 of secretion 40 penetrates microporous membrane 34 to react with indicator substrate 36. Panty shield 32 in Figure 3 further includes two side flaps 46 (only one is visible in Figure 3) configured to allow attachment of panty shield 32 to an undergarment of a user, in such a way keeping panty shield 32 in the proximity of the vagina of a user.

In a second method of attaching an indicator to a substrate , a substrate is first treated with a surfactant solution. After the solution dries, an indicator solution is applied to the substrate. The substrate can then be integrated into a product.

Although any surfactant can be used, when it is desired to attach negatively charged indicators to a neutral substrate, a surfactant with a cationic functional group is used, preferably Aliquat 336, TDMAC or CTAC. Although any suitable solvent or mixture of solvents may be used, preferred are ethyl acetate or substantially volatile ethers such as diethyl ether, isopropyl ether, t-butyl methyl ether or tetrahydrofuran. The surfactant is dissolved in the solvent at any suitable concentration. Preferred is that the surfactant makes up 0.01% to 2% of the solution, more preferred is that the surfactant makes up 0.1% to 0.5% of the solution, and most preferred is that the surfactant makes up 0.15% to 0.25% by weight of the solution. As is clear to one skilled in the art, it is also possible to use a combination of suitable surfactants. The surfactant solution is applied to the substrate. Application is done, for example, by spraying or spreading the mixture on the substrate, or by dipping the substrate in the mixture. The substrate can be of many suitable materials known in the art such as polyester membranes, polypropylene membranes, cellulose membranes, paper, cotton or linen. The structure of the substrate may be, for example, a fiber, a mesh, gauze, a fabric or a membrane. The solvent of the surfactant solution is allowed to evaporate. Although the solvent may be allowed to evaporate at ambient pressure, it is preferable to evaporate the solvent under vacuum, preferably at a pressure of less than 600 mm Hg, more preferably less than 200 mm Hg, and even more preferably less than 100 mm Hg.

After the solvent of the surfactant solution has evaporated, an indicator solution is applied to the substrate. Although any solvent or mixture of solvents may be used, preferred are ethyl acetate or substantially volatile ethers such as diethyl ether, isopropyl ether, t-butyl methyl ether, or tetrahydrofuran. Although any suitable indicator can be used, it is preferred that the indicator molecules have a negatively charged functional group such as acetate or sulfonate when the surfactant used is a cationic surfactant. Most preferably, the indicators used, separately or in combination are chosen from amongst those listed in Table 1 or, for example, in US Patent 5,897.834. The amount of indicator added is 0.00001 % to 1 % of the weight of the indicator solution as described above. Preferred is that the indicator is 0.0001% to 0.1% of the indicator solution, more preferred is that the indicator is 0.001 % to 0.01% of the indicator solution, and most preferred is that the indicator is 0.002% to 0.004% of weight of the indicator solution.

When it is desired to add a reagent in preparation of the indicator system of the present invention, reagent is added to the indicator solution. For example, when urease is used, any suitable amount of urease can be added. Although any suitable concentration of urease can be used, preferred is a concentration of between 1 and 100 unit/ml. More preferred is a concentration of 2 and 50 unit/ml and even more preferred a concentration of 5 and 20 unit/ml.

In an additional embodiment of the present invention, a reagent solution is prepared separately from the indicator solution. When urease is used as a reagent, any suitable concentration of urease can be used. It is preferred that a concentration of between 1 and 100 unit/ml urease be used, more preferred is a concentration of 2 and 50 unit/ml and even more preferred a concentration of 5 and 20 unit/ml.

The indicator solution (or indicator / reagent solution) is applied to the substrate. Application can be done, for example, by spraying or spreading the indicator solution on the substrate, or by dipping the substrate in the indicator solution. The solvent of the indicator solution is allowed to evaporate. Although the solvent may be allowed to evaporate at ambient pressure, it is preferable to evaporate the solvent under vacuum, preferably at a pressure of less than 600 mm Hg, more preferably less than 200 mm Hg, and even more preferably less than 100 mm Hg.

When a reagent solution is prepared separately from the indicator solution, the reagent solution is applied in substantially the same way as described hereinabove, either before or after application of the indicator solution.

Irrespective of the exact concentration of the indicator solution and of the surfactant solution used, it is preferable to apply an amount of each one of the solutions so that the molar concentration of surfactant applied per unit area of substrate is roughly one hundred times greater than the molar concentration of indicator applied per unit area of substrate. The indicator solution is applied to the substrate to areas where surfactant was previously applied.

As is clear to one skilled in the art, when the indicator system of the present invention is implemented, a first solution with a first indicator is made, and a second solution with a second indicator and a reagent is made, both solutions as described hereinabove. Each of the two solutions is applied in distinct areas on the substrate, as described hereinabove.

**EXAMPLE 5:**

Solution A: 370 mg cellulose acetate, 280 mg DBP, 150 mg sorbitol, 150 mg 2-ethoxyethanol, 50 mg TDMAC wee combined. 3 mg Bromthymol blue were added, 20 ml THF were added. The solution was vigorously stirred.

Solution B: 370 mg cellulose acetate, 280 mg BBPA, 300 mg ethylene glycol, 50 mg TDMAC were combined. 3 mg m-cresol purple and 30 units urease were added. 20 ml 20 THF were added. The solution was vigorously stirred.

1a. Cotton gauze was dipped in Solution A. When the solution dried, the cotton gauze was cut in half. The first half was dipped in a pH 7 test solution. The first half became purple. The first half was allowed to dry in ambient conditions, with no substantial change of color. After three hours, the second half was dipped in a pH 7 test solution. The second half became purple. The colors of the first half and of the second half were substantially the same.

1b. Cotton gauze was dipped in Solution B. When the solution dried, the cotton gauze was cut in half. The first half was dipped in urine. The first half became violet. The first half was allowed to dry in ambient conditions, with no substantial change of color. After three hours, the second half was dipped in urine. The second half became violet. The colors of the first half and of the second half were substantially the same.

1c. Solution A and Solution B were applied in alternating stripes on cotton gauze at a density of about 50 ul/mm2. Amniotic fluid was applied to the gauze, changing the color of the stripes of Solution A to purple. Urine was applied to the gauze, changing the color of the stripes of Solution B to violet. The gauze was allowed to dry at ambient conditions for three hours and cut in half. Urine was applied to the first half. The colors of the stripes in the first half and the second half of the gauze were substantially the same.

**EXAMPLE 6:**

Three solutions were prepared:

Solution A: 0.2% Aliquot 336 in DDW (double distilled water);
Solution B: 10 unit/ml urease and 0.003% m-cresol purple in DDW; and
Solution C: 0.003% nitrazine yellow in isopropyl ether.

A nitrocellulose membrane was dipped in Solution A and transferred to an atmosphere of 50 mm Hg. After 30 minutes, the membrane was removed from the vacuum. Solution B was applied in a pattern resembling the word "NO" at a density of 50 μl / mm2. Solution C was applied in a pattern resembling the word "AMNIO" at a density of 50 μl / mm2. The membrane was transferred to an atmosphere of 50 mm Hg. After 30 minutes, the membrane was removed from the vacuum. The membrane was dipped in a pH 7 test solution. The word AMNIO appeared in purple. After drying at ambient conditions for three hours, no substantial change of color was observed. The membrane was dipped in urine. The word NO appeared in violet.

It is clear to one skilled in the art that the present invention is not limited to the embodiments described herein but also relates to many types of conventional modifications thereof, which are within the scope of the claims.

## Method of constructing the article

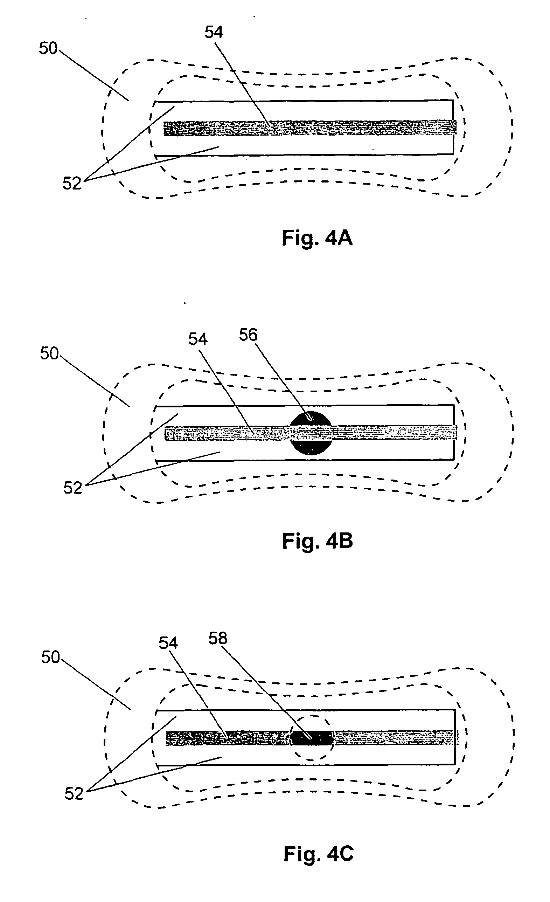

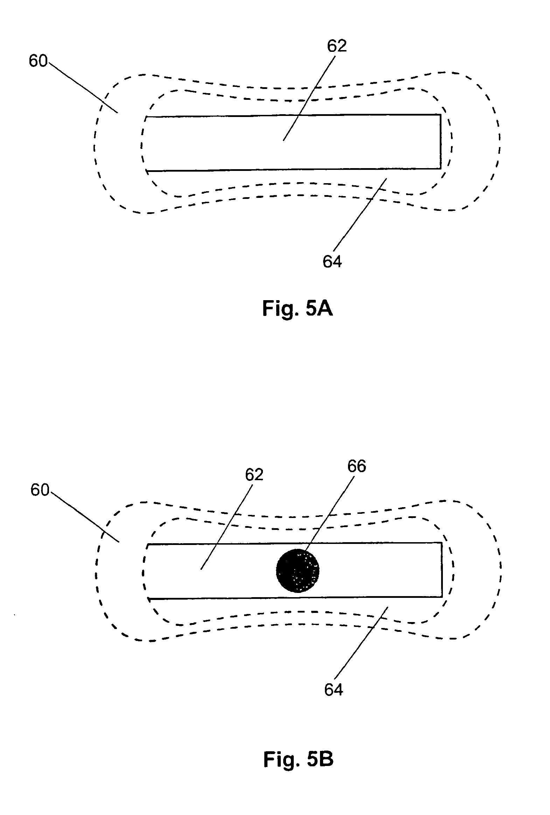

Figures 4A-C and 5A-B provide visual examples of two methods non-limiting examples of constructing the article. Figures 4A-C show an article in the form of a panty shield 50 constructed with two indicators 52 and 54. In Figure 4A, the panty shield 50 is constructed with a pH indicator 52 for detecting normal biological fluids and a second pH indicator with high pH dye and a reagent, such as urease, for detecting interfering biological fluids, such as urine. Figure 4B depicts the panty shield 50 wherein a normal fluid, without an interfering fluid, changes the color 56 of the pH indicator 52. In contrast, Figure 4C depicts the panty shield 50, wherein an interfering biological fluid , such as urine, changes the color 58 of the second pH indicator 54.

In a separate embodiment, the article can be made with only a single indicator as shown in Figures 5A and 5B. Figure 5A depicts the article in the form of a panty shield 60, comprising a sticker 64, with an indicator 62 constructed so as not to react with an interfering biological fluid like urine. When a biological fluid to be detected comes in contact with the indicator, as show in Figure 5B, the indicator changes color 66, whereas if the indicator comes in contact with urine it will not change colors.

## Claims

1. A secretion-monitoring article for the identification of secreted biological fluids comprising: a body that includes an absorbent material for absorbing a biological fluid secreted from a person and an indicator system comprising at least one pH determining member and an ion-balance reagent for reacting with the biological fluid, wherein the indicator system provides an indication of health conditions associated with the pH, protonated amine cation and buffer capacities of the biological fluids, and wherein the indicator system is associated with the absorbent material such that the biological fluids contact the indicator system.

2. The article of claim 1, wherein the indication is resistant to change due to long use or wetting and drying cycles.

3. The article of claim 1, further comprising a plurality of pH determining members.

4. The article of claim 1, further comprising a reagent that substantially changes color upon contact with urine.

5. The article of claim 4, wherein the reagent is urease.

6. The article of claim 1, further comprising a mounting means for placing the absorbent body in a position to receive biological fluids secreted from the person.

7. The article of claim 1, wherein the absorbent material of the body is a swab, gauze, panty shield, hygienic napkin, a diaper or interlabial absorbent structure.

8. The article of claim 1, wherein the indicator system comprises a first pH indicator attached to the substrate in a first area and having a color transition at a first pH range; a second pH indicator attached to the substrate in a second area and having a color transition at a second pH range; and a reagent attached to the substrate in the second area, wherein the first pH range and the second pH range are different and wherein said reagent substantially changes color upon contact with urea.

9.  The article of claim 8, wherein the first area and the second areas are separated.

10. The article of claim 8, comprising a microporous membrane for the secretion absorbed by the body through which the biological fluid must pass prior to absorption by the absorbent material.

11. The article of claim 8, wherein the first pH indicator is configured to substantially change color upon contact with amniotic fluid or with vaginal secretions associated with vaginosis and the second pH indicator is configured to substantially change color upon contact with urine reacting with the reagent.

12. The article of claim 8, wherein the first and second pH indicators each have a negatively charged functional group.

13. The article of claim 8, wherein the first pH range is lower than the second pH range.

14. The article of claim 8, further comprising a third pH indicator.

15. A method for monitoring the health condition of a person comprising: positioning the article of claim 1 to absorb fluids secreted from the vicinity of the vaginal area of a female; and inspecting the pH indicator in order to identify the type of secretion contacting the pH indicator.

16. The article according to claim 1 for the identification of infected urine, wherein the indicator system reacts with normal urine differently than with infected urine, and wherein the color change obtained upon contact between the indicator system and infected urine is nonreversible.

17. The article according to claim 1 for the identification of bacterially infected vaginal secretions, wherein the indicator system reacts with normal or candida vaginal secretions differently than with bacterially infected vaginal secretions.

18. The article of claim 1, wherein the indicator system <u>further</u> comprises: a pre-formed polymer in an amount of about 20 to 50% of the total weight; a plasticizer polymer in an amount of about 15 to 40% of the total weight; a wetting agent polymer in an amount of about 15 to 45% of the total weight; an ion-balance reagent polymer in an amount of about 0.1 to 10% of the total weight; and an indicator polymer in an amount of about 0.05 to 5% of the total weight, wherein the percents are weight percents based on the total weight of the mixture and the total weight of the mixture equals 100%.

19. The article of claim 18, further comprising a reagent in an amount of about 10 units for each 0.01 to 0.1 % of indicator in the mixture.

20. The article of claim 19, wherein the reagent is urease.

21. The article of claim 18, further comprising a solvent in an amount of about 1 to 30 ml for each 150 mg of mixture.

22. The article of claim 18, wherein the pre-formed polymer is present in an amount of about 36 to 39%, the plasticizer is present in an amount of about 27 to 29%, the wetting agent is present in an amount of about 29 to 31% the ion-balance reagent is present in an amount of about 4 to 6%, the indicator is present in an amount of 0.2 to 0.4%.

**Patentansprüche**

1.  Artikel zur Sekretionsbeobachtung für die Identifizierung sekretierter biologischer Flüssigkeiten, umfassend: einen Körper, der ein Absorptionsmaterial zur Absorption einer von einem Individuum sekretierten biologischen Flüssigkeit umfasst, und ein Indikatorsystem, das mindestens ein pH bestimmendes Element und ein Reagenz zum Ionenausgleich zur Reaktion mit der biologischen Flüssigkeit umfasst, worin das Indikatorsystem eine Anzeige von Gesundheitszuständen bereitstellt, die mit dem pH, protoniertem Aminkation und den Pufferkapazitäten der biologischen Flüssigkeiten zusammenhängen, und worin das Indikatorsystem mit dem Absorptionsmaterial derart zusammenhängt, dass die biologischen Flüssigkeiten das Indikatorsystem kontaktieren.

2.  Artikel nach Anspruch 1, worin die Anzeige gegenüber langem Gebrauch oder Benetzungs- und Trocknungszyklen änderungsbeständig ist.

**3.** Artikel nach Anspruch 1, ferner mehrere pH bestimmende Elemente umfassend.

**4.** Artikel nach Anspruch 1, ferner ein Reagenz umfassend, das bei Kontakt mit Urin im Wesentlichen die Farbe ändert.

**5.** Artikel nach Anspruch 4, worin das Reagenz Urease ist.

**6.** Artikel nach Anspruch 1, ferner ein Befestigungsmittel zum Platzieren des Absorptionskörpers in einer Lage umfassend, um von dem Individuum sekretierte biologische Flüssigkeiten aufzunehmen.

**7.** Artikel nach Anspruch 1, worin das Absorptionsmaterial des Körpers ein Tupfer, Gaze, eine Slipeinlage, Hygieneeinlage, eine Windel oder interlabiale Absorptionsstruktur ist.

**8.** Artikel nach Anspruch 1, worin das Indikatorsystem einen ersten pH Indikator, der an dem Substrat in einem ersten Bereich angebracht ist und einen Farbübergang in einem ersten pH Bereich aufweist; einen zweiten pH Indikator, der an dem Substrat in einem zweiten Bereich angebracht ist und einen Farbübergang in einem zweiten pH Bereich aufweist; und ein Reagenz umfasst, das an dem Substrat in dem zweiten Bereich angebracht ist, worin der erste pH Bereich und der zweite pH Bereich verschieden sind und worin das Reagenz bei Kontakt mit Urin im Wesentlichen die Farbe ändert.

**9.** Artikel nach Anspruch 8, worin der erste Bereich und der zweite Bereich getrennt sind.

**10.** Artikel nach Anspruch 8, eine mikroporöse Membran für die durch den Körper absorbierte Sekretion umfassend, durch die die biologische Flüssigkeit vor Absorption durch das Absorptionsmaterial passieren muss.

**11.** Artikel nach Anspruch 8, worin der erste pH Indikator ausgestaltet ist, im Wesentlichen die Farbe bei Kontakt mit Fruchtwasser oder mit Scheidensekreten zu ändern, das/die mit Vaginose zusammenhängt/zusammenhängen, und der zweite pH Indikator ausgestaltet ist, im Wesentlichen die Farbe bei Kontakt mit Urin zu ändern, der mit dem Reagenz reagiert.

**12.** Artikel nach Anspruch 8, worin der erste und zweite pH Indikator jeweils eine negative geladene, funktionelle Gruppe aufweist.

**13.** Artikel nach Anspruch 8, worin der erste pH Bereich kleiner als der zweite pH Bereich ist.

**14.** Artikel nach Anspruch 8, ferner einen dritten pH Indikator umfassend.

**15.** Verfahren zur Beobachtung des Gesundheitszustands eines Individuums, umfassend: Positionieren des Artikels nach Anspruch 1 zur Absorption von Flüssigkeiten, die von der Umgebung des Vaginalbereichs einer Frau sekretiert werden; und Prüfen des pH Indikators, um die Art der den pH Indikator kontaktierenden Sekretion zu bestimmen.

**16.** Artikel nach Anspruch 1 für die Identifizierung von infiziertem Urin, worin das Indikatorsystem mit gewöhnlichem Urine unterschiedlich als mit infiziertem Urin reagiert und worin die Farbänderung, die bei Kontakt zwischen dem Indikatorsystem und infiziertem Urin erreicht wird, irreversibel ist.

**17.** Artikel nach Anspruch 1 für die Identifizierung von bakteriell infizierten Scheidensekreten, worin das Indikatorsystem mit gewöhnlichen oder Candida-Scheidensekreten verschieden als mit bakteriell infizierten Scheidensekreten reagiert.

**18.** Artikel nach Anspruch 1, worin das Indikatorsystem ferner umfasst: ein vorgebildetes Polymer in einer Menge von ungefähr 20 bis 50% des Gesamtgewichts; ein Weichmacherpolymer in einer Menge von ungefähr 15 bis 40% des Gesamtgewichts; ein Netzmittelpolymer in einer Menge von ungefähr 15 bis 45% des Gesamtgewichts; ein Polymerreagenz zum Ionenausgleich in einer Menge von ungefähr 0,1 bis 10% des Gesamtgewichts; und ein Indikatorpolymer in einer Menge von ungefähr 0,05 bis 5% des Gesamtgewichts, worin die Prozentangaben Gewichtsprozente beruhend auf dem Gesamtgewicht des Gemischs sind und das Gesamtgewicht des Gemischs 100% entspricht.

**19.** Artikel nach Anspruch 18, ferner ein Reagenz in einer Menge von ungefähr 10 Einheiten für jedes 0,01 bis 0,1% an Indikator in dem Gemisch umfassend.

**20.** Artikel nach Anspruch 19, worin das Reagenz Urease ist.

**21.** Artikel nach Anspruch 18, ferner ein Lösungsmittel in einer Menge von ungefähr 1 bis 30 ml für jeweils 150 mg Gemisch umfassend.

**22.** Artikel nach Anspruch 18, worin das vorgebildete Polymer in einer Menge von ungefähr 36 bis 39% vorliegt, der Weichmacher in einer Menge von ungefähr 27 bis 29% vorliegt, das Benetzungsmittel in einer Menge von ungefähr 29 bis 31% vorliegt, das Reagenz zum Ionenausgleich in einer Menge von ungefähr 4 bis 6% vorliegt, der Indikator in einer Menge von ungefähr 0,2 bis 0,4% vorliegt.


**Revendications**

**1.** Un article de contrôle de sécrétion pour l'identification de liquides biologiques secrétés comprenant : un corps incorporant un matériau pour absorber un liquide biologique sécrété par une personne et un système indicateur comprenant au moins un élément déterminant le pH et un réactif de balance ionique pour réagir avec le fluide biologique, dans lequel système indicateur fournit une indication des conditions de santé associées avec le pH, le cation amine protonée et les capacités de de tampon des liquides biologiques et dans lequel le système indicateur est associé à un matériau absorbant de sorte que les liquides biologiques viennent au contact du système indicateur.

**2.** L'article de la revendication 1 dans lequel l'indication est résistante aux changements dus à un usage prolongé ou aux cycles d'humidification et de séchage.

**3.** L'article de la revendication 1 comprenant en outre une pluralité d'éléments de détermination du pH.

**4.** L'article de la revendication 1 comprenant en outre un réactif qui change substantiellement de couleur par contact avec l'urine.

**5.** L'article de la revendication 4 dans lequel le réactif est l'uréase.

**6.** L'article de la revendication 1 comprenant en outre des moyens de montage pour mettre en place le corps absorbant en position de recevoir les fluides biologiques sécrétés par la personne.

**7.** L'article de la revendication 1 dans lequel le matériau absorbant du corps est un tampon, une gaze, un protège slip, une serviette hygiénique, une couche-culotte ou une structure absorbante interlabiale.

**8.** L'article de la revendication 1 dans lequel le système indicateur comprend un premier indicateur de pH attaché au substrat dans une première zone et comportant une transition de couleur pour un premier intervalle de pH, un second indicateur de pH attaché au substrat dans une seconde zone du substrat et comportant une transition de couleur pour un second intervalle de pH, dans lequel le premier intervalle de pH et le second intervalle de pH sont différents et dans lequel ledit réactif change substantiellement de couleur par contact avec l'urée.

**9.** L'article de la revendication 8 dans lequel les première et seconde zones sont séparées.

**10.** L'article de la revendication 8 comprenant une membrane microporeuse pour la sécrétion absorbée par le corps, au travers de laquelle le liquide biologique doit passer avant absorption par le matériau absorbant.

**11.** L'article de la revendication 8 dans lequel le premier indicateur de pH est configuré pour changer substantiellement de couleur par contact avec du liquide amniotique ou des sécrétions vaginales associées à la vaginose et le second indicateur de pH est configuré pour changer substantiellement de couleur par contact avec l'urine réagissant avec le réactif.

**12.** L'article de la revendication 8 dans lequel les premier et second indicateurs de pH comportent chacun un groupe fonctionnel chargé négativement.

**13.** L'article de la revendication 8 dans lequel le premier intervalle de pH est inférieur au second intervalle de pH.

**14.** L'article de la revendication 8 dans comprenant un troisième indicateur de pH.

**15.** Un procédé de contrôle des conditions de santé d'une personne comprenant : placer l'article de la revendication 1 pour absorber les liquides sécrétés à proximité de la zone vaginale de la femme et inspecter l'indicateur de pH afin d'identifier le type de sécrétion contactant l'indicateur de pH.

**16.** L'article de la revendication 1 pour l'identification d'urine infectée dans lequel le système indicateur réagit avec l'urine normale différemment d'avec l'urine infectée et dans lequel le changement de couleur obtenu par contact entre le système indicateur et l'urine infectée est irréversible.

**17.** L'article de la revendication 1 pour l'identification de sécrétions vaginales infectées dans lequel le système indicateur réagit avec des sécrétions vaginales ou de candida normales différemment d'avec des sécrétions vaginales infectées par des bactéries.

**18.** L'article de la revendication 1 dans lequel le système indicateur comprend en outre : un polymère préformé en une quantité d'environ 20 à 50 % du poids total, un plastifiant polymère en une quantité d'environ 15 à 40 % du poids total, un agent mouillant polymère en une quantité d'environ 15 à 45 % du poids total, un réactif de balance ionique polymère en une quantité d'environ 0.1 à 10 % du poids total et un indicateur polymère en une quantité d'environ 0.05 à 5 % du poids total, dans lequel les pour cent en poids sont basés sur le poids total du mélange et le poids total du mélange est égal à 100 %.

**19.** L'article de la revendication 18 comprenant en outre un réactif en une quantité d'environ 10 unités pour chaque 0.01 à 0.1 % d'indicateur dans le mélange.

**20.** L'article de la revendication 19 dans lequel le réactif est l'uréase.

**21.** L'article de la revendication 18 comprenant en outre un solvant en une quantité d'environ 1 à 30 ml pour chaque 150 g de mélange.

**22.** L'article de la revendication 18 dans lequel le polymère préformé est présent en une quantité d'environ 36 à 39 %, le plastifiant est présent en une quantité d'environ 27 à 29 %, l'agent humidifiant est présent en une quantité d'environ 29 à 31 %, le réactif de balance ionique est présent en une quantité d'environ 4 à 6 %, l'indicateur est présent en une quantité d'environ 0.2 à 0.4 %

**Fig. 1A**

Fig. 1B

10

22

14

16

NO
AMNIO

12

16

NO
AMNIO

12

Fig. 1C

**Fig. 2**

**Fig. 3**

**Fig. 4A**

**Fig. 4B**

**Fig. 4C**

**Fig. 5A**

**Fig. 5B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5217444 A
- US 5823953 A
- US 6106461 A
- WO 0113097 A
- US 6126597 A
- US 6149590 A
- US 5897834 A

### Non-patent literature cited in the description

- **E. BISHOP.** Indicators. Pergamon Press, 1972